# EUROPÄISCHE PATENTSCHRIFT

(11) **EP 0 452 451 B1**
(45) Veröffentlichungstag und Bekanntmachung des Hinweises auf die Patenterteilung: **12.06.1996**
(21) Anmeldenummer: 90916209.1
(22) Anmeldetag: 02.11.1990
(51) Int. Cl.: A61B 17/60, A61B 17/70

(54) **PEDIKELSCHRAUBE UND KORREKTUR- UND HALTEVORRICHTUNG MIT EINER SOLCHEN PEDIKELSCHRAUBE**
PEDICLE SCREW, AND CORRECTION AND RETAINING DEVICE WITH SAID PEDICLE SCREW
VIS POUR PEDICULE VERTEBRAL ET DISPOSITIF DE CORRECTION ET DE MAINTIEN POURVU D'UNE TELLE VIS

(30) Priorität: 03.11.1989 DE 3936702
(43) Veröffentlichungstag der Anmeldung: 23.10.1991
(73) Patentinhaber: Biedermann, Lutz, 78048 Villingen-Schwenningen (DE); Harms, Jürgen, Prof. Dr., 76337 Waldbronn (DE)
(72) Erfinder: Biedermann, Lutz, 78048 Villingen-Schwenningen (DE); Harms, Jürgen, Prof. Dr., 76337 Waldbronn (DE)
(74) Vertreter: Prüfer, Lutz H., Dipl.-Phys.
(86) Internationale Anmeldenummer: EP9001834
(87) Internationale Veröffentlichungsnummer: WO9106254

(56) Entgegenhaltungen:
- EP-A- 0 140 786
- EP-A- 0 242 708
- DE-A- 3 639 810
- US-A- 4 836 196

## Beschreibung

Die Erfindung betrifft eine Pedikelschraube, die insbesondere zusammen mit einer Korrektur- und Haltevorrichtung für die Wirbelsäule einsetzbar ist, sowie eine Korrektur- und Haltevorrichtung für die Wirbelsäule mit einer solchen Pedikelschraube.

Aus Unfallchirurgie, Heft 12 (1986), Seiten 68 - 79 ist das mechanische Prinzip zur dorsalen Stabilisierung der Brust- und Lendenwirbelsäule mit Hilfe einer Korrektur- und Haltevorrichtung bekannt. Aus einem Prospekt "Fixateur Interne für die Wirbelsäule, Original-Instrumente und -Implantate der Schweizerischen Arbeitsgemeinschaft für Osteosynthesefragen-AO" ist ein Fixateur Interne bekannt. Dieser umfaßt zwei Paare von sogenannten Schanz'schen Schrauben. Von diesen wird ein erste Paar durch die beidseitigen Bogenwurzeln in den ersten Wirbelkörper und ein zweites Paar durch die beidseitigen Bogenwurzeln in einen zweiten Wirbelkörper eingeschraubt. Die Schanz'schen Schrauben stehen mit einem ungefähr 10 cm langen Schaft dann aus den Wirbelkörpern hervor. Nach dem Einbringen der vier Schanz-Schrauben wird mittels der langen, vorstehenden Schraubenenden bereits manuell eine partielle Reposition der Wirbel möglich. Anschließend werden Gewindestäbe auf jeweils zwei auf der gleichen Seite der übereinanderliegenden Wirbel befindlichen Schanz-Schrauben aufgesteckt. Dann erfolgt erneut eine Einwirkung auf die hervorstehenden freien Enden der Schanz'schen Schrauben und ein Arretieren der Gewindestäbe. Abschließend werden die über die Gewindestäbe überstehenden Teile der Schanz-Schrauben abgeschnitten. Dann wird die Muskulatur und Haut über den Fixateur gelegt, und die Wunde wird geschlossen. Stellt der Operateur am nächsten Tag fest, daß eine Veränderung der Winkelstellung der Wirbel erforderlich wäre, ist dies nicht mehr möglich, da die Ausrichtung der Winkelstellung erforderlichen überstehenden Teil der Schanz-Schrauben bereits entfernt sind. Ferner ist die Rotationsfertigkeit der Vorrichtung gering.

Aus der DE 36 39 810 A1 ist ein Implantat zur Wirbelsäulenkorrektur und/oder Stabilisierung bekannt. Das Implantat weist Schrauben mit einem Gewindeschaftteil und einem diesem abgewandten und mit dem Gewindeschaftteil starr verbundenen Aufnahmeteil auf. Jeweils ein Schraubenpaar ist an seinem einen Ende über das jeweilige Aufnahmeteil mit einer Spannstange starr verbunden. Das Schraubenpaar ist ferner an einer Stelle der Schrauben, die einen Abstand von der Verbindung zwischen Aufnahmeteil und Spannstange hat, zusätzlich mit einem Spannstab verbunden. Damit die axiale Richtung der Schrauben und damit der diese aufnehmenden Wirbelkörper von der Parallelstellung abweichend ausgerichtet werden kann, wird die Verbindung zwischen Spannstab und den beiden Schrauben so verstellt, daß auf die Spannstange oder den Spannstab eine Biegeverformung ausgeübt wird. Damit erfolgt eine unkontrollierbare Materialbeanspruchung, die im Laufe der Zeit zu einer Zerstörung der Spannstange und/oder des Spannstabes führen kann, was im Körperinneren natürlich zu nicht vorhersehbaren Problemen führen kann.

Es wurde gefunden, daß beim Aufspreizen benachbarter Wirbel mit einer solchen Korrektur- und Haltevorrichtung aufgrund der vorhandenen Bänder der Wirbel, der zwischen denjenigen liegt, in die die Schrauben der Korrektur- und Haltevorrichtung eingreifen, in die Richtung versetzt wird, in der die aufgespreizten Wirbel näher beieinander liegen, was natürlich ein unerwünschter Effekt ist.

Aus US-A-4 836 196 ist eine Pedikelschraube mit einem Gewindeschaftteil bekannt. Diese dient zum Verbinden mit einer Schiene. Der Schraubenkopf ist als Gewindestange ausgebildet. Zum Verbinden weist die Schiene Bohrungen zum Aufschieben auf die Gewindestange auf. Konzentrisch zur Bohrung ist die Oberfläche der Schiene hohlkugelsegmentförmig ausgebildet. Zum Verbinden zwischen Schraube und Schiene werden zwischen dem Schraubenkopf und der Schiene auf der einen Seite und zwischen der Schiene und einer Feststellmutter auf der anderen Seite kugelsegmentförmige Unterlegelemente aufgesetzt, die mit den hohlkugelsegmentförmigen Bereichen zum Erzielen einer festen Verbindung zusammenwirken.

Aus der EP-A-0 242 708 ist eine Pedikelschraube mit den Merkmalen des Oberbegriffes des Patentanspruchs 1 bekannt. Die Pedikelschraube weist ein kopfseitig vorgesehenes Aufnahmeteil für eine Stange auf, wobei das Aufnahmeteil mit dem Gewindeschaftteil gelenkig verbunden ist.

Aufgabe der Erfindung ist es, eine Pedikelschraube, die zum Ausgleich im Zusammenwirken mit einer Korrektur- und Haltevorrichtung im Wirbelsäulenbereich anwendbar ist, sowie eine Korrektur- und Haltevorrichtung insbesondere für die Wirbelsaule unter Verwendung einer solchen Pedikelschraube, mit der das Versetzen eines Wirbels quer zur Wirbelsauleneinrichtung bei Anwenden der Korrektur- und Haltevorrichtung verhindert bzw. ausgeglichen wird, zu schaffen. Diese Aufgabe wird durch die in Patentanspruch 1 beschriebene Pedikelschraube gelost.

Eine Korrektur- und Haltevorrichtung zur Losung der Aufgabe ist in Patentanspruch 6 gekennzeichnet.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht der Korrektur- und Haltevorrichtung, teilweise in geschnittener Darstellung;
- Fig. 2: eine Draufsicht auf die in Fig. 1 gezeigte Vorrichtung;

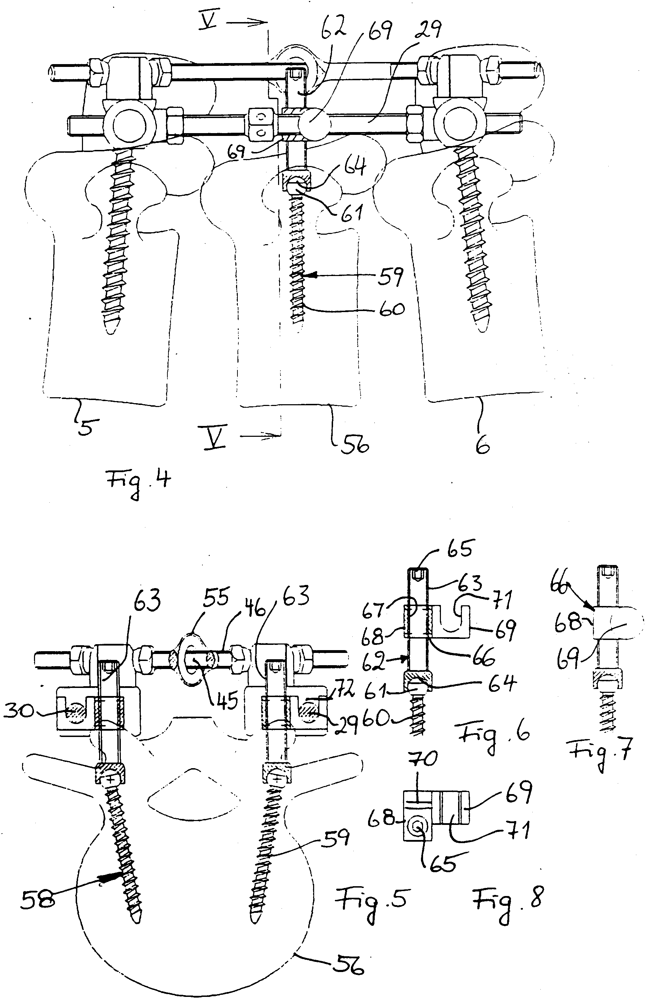

- Fig. 3: einen Schnitt entlang der Linie III/III in Fig. 1;
- Fig. 4: eine Darstellung ähnlich der in Fig. 1, mit Pedikelschraube;
- Fig. 5: einen Schnitt entlang der Linie V/V;
- Fig. 6: eine Seitenansicht der Pedikelschraube, teilweise in geschnittener Darstellung;
- Fig. 7: eine gegenüber der in Fig. 6 gezeigten Darstellung um 90 ° verdrehte Seitenansicht der Pedikelschraube;
und
- Fig. 8: eine Draufsicht auf die Pedikelschraube.

Die Korrektur- und Haltevorrichtung unfaßt vier Schrauben 1-4 als Knochenschrauben. Das erste Paar Schrauben 1,3 dient beim Einsetzen der Korrektur- und Haltevorrichtung zur Stabilisierung der Wirbelsäule zum Einschrauben in die Bogenwurzeln in einen Wirbelkörper 5, der schematisch angedeutet ist. Das zweite Paar Schrauben 2, 4 dient entsprechend zum Einschrauben in die Bogenwurzeln eines zweiten Wirbelköpers 6, der ebenfalls schematisch angedeutet ist.

Jede der Schrauben 1-4 umfaßt einen Gewindeschaftteil 7 mit einem Gewindeteil 9 und einem am kopfseitigen Ende des Gewindeteiles. vorgesehenen kugelsegmentförmigen Kopf 10 sowie einen Aufnahmeteil 8. Der Kopf weist auf seiner dem Gewindeteil 9 abgewandten Seite eine sich senkrecht zur Achse des Gewindeteiles erstreckende ebene Fläche 11 auf. Koaxial zu dem Gewindeteil weist die ebene Fläche einen Schlitz oder eine andere Eingriffsmöglichkeit für einen Schraubendroher zum Einschrauben der Schraube in einen Wirbelkörper auf.

Das Aufnahmeteil 8 umfaßt zwei Kopfhälften 12,13 sowie einen diese zusammenhaltenden Haltering 14. Jede Kopfhälfte weist auf ihrer der anderen Kopfhälfte zugewandten Innenseite einen hohlkugelsegmentförmigen Abschnitt auf, dessen Innenradius dem Außenradius des kugelsegmentförmigen Kopfes 10 entspricht. An den kugelsegmentförmigen Abschnitt schließt sich ein Halsabschnitt 15 an. Dieser hat die Form eines Segmentes eines Hohlkugelabschnittes und ist von dem kugelsegmentförmigen Abschnitt ausgehend nach außen divergent ausgebildet. Die Achse des Halsabschnittes geht durch den Mittelpunkt des hohlkugelsegmentförmigen Abschnittes. Auf der dem Halsabschnitt gegenüberliegenden Seite des kugelsegmentförmigen Abschnittes erstreckt sich senkrecht zu der Symmetrieachse von Halsabschnitt und kugelsegmentförmigen Abschnitt ein Aufnahmeschlitz 16, in dessen der anderen Kopfhälfte abgewandten Außenseite eine Versenkung 17 vorgesehen ist. Die Breite des Aufnahmeschlitzes 16 ist so gewählt, daß eine aufzunehmende Gewindestange lose durch diesen hindurchführbar ist. Auf diese Weise ist der Aufnahmeteil innerhalb eines Kegelwinkels um die Achse des Gewindeschaftteiles 7 frei schwenkbar und um die Achse der Schraube zum Ausrichten der Schlitzrichtung frei drehbar.

Zwischen dem Aufnahmeteil 8 und dem Gewindeteil 9 weist jede der Schrauben ein sich senkrecht zur Schraubenachse erstreckendes Zylinderstuck 18-21 auf. An einem Ende des Zylinderstückes erstreckt sich senkrecht zur Zylinderachse eine Bohrung 23 durch dieses hindurch, deren Durchmesser so gewählt ist, daß ein Hals 22 der jeweiligen Schraube zwischen Kopf und Gewindeteil gerade in die Bohrung einsetzbar ist. Auf der anderen Seite des Zylinderstückes ist koaxial zu seiner Achse eine einen Bolzen 24 aufnehmende Bohrung 25 vorgesehen. Der Bolzen 24 wiest senkrecht zu der Achse der Bohrung und damit zu der Zylinderachse eine Gewindebohrung auf. Die Abmessungen des Bolzens sind so gewählt, daß er in der Bohrung 25 frei um seine Achse drehbar ist. Senkrecht zu der Bohrung 23 und senkrecht zu der Bohrung 25 ist eine weitere Bohrung 26 in dem Zylinder angebracht. Diese ist koaxial zu der Gewindebohrung im Bolzen angeordnet, so daß eine Schraube durch diese hindurch in den Bolzen 24 einschraubbar ist. Die beiden Austrittsenden der Bohrung 26 weisen in einer Richtung parallel zur Schraubenachse kegelförmige Aufweitungen 27,28 auf, die ein Verschwenken der aufzunehmenden Schrauben innerhalb eines durch die Größe der kegelförmigen Aufweitung bestimmten Bereiches um die Längsachse des Zylinderstückes ermöglichen.

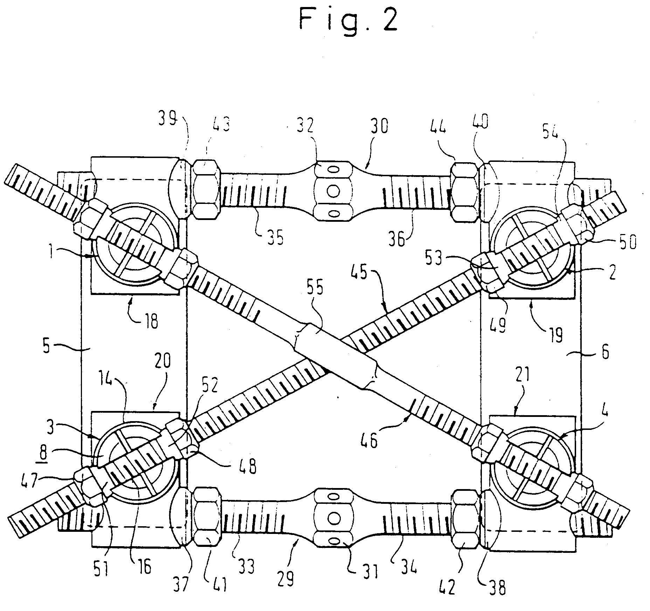

Die Korrektur- und Haltevorrichtung weist ferner ein Paar Gewindestangen 29,30 auf. Diese besitzen in einem zentralen Abschnitt einen koaxial ausgerichteten Sechskantteil 31,32 zum Ineingriffbringen mit einem Schraubschlüssel und beidseitig daran anschließende Gewindebereiche 33,34; 35,36. Die jeweiligen gegenüberliegenden Gewindebereiche 33,34 bzw. 35,36 einer Gewindestange weisen in ihrer Drehrichtung einander entgegengesetzte Rechts- bzw. Links-Gewinde auf. Für jeden Gewindeabschnitt ist ein quasi als Unterlegscheibe wirkendes Zwischenglied 37-40 vorgesehen, welches so ausgebildet ist, daß es auf seiner dem Sechskant zugewandten Seite eine ebene Fläche und auf seiner gegenüberliegenden Seite eine hohlzylindersegmentförmige Oberfläche aufweist. Letztere entspricht in ihrer Krümmung der Krümmung der Außenorberfläche der jeweiligen Zylinderstücke 18-21. Zwischen Sechskantteil und Zwischenglied ist jeweils eine Mutter 41-44 vorgesehen. Wie am besten aus Fig. 2 ersichtlich ist, ist eine Gewindestange 29 mit den beiden auf einer Seite der Wirbelsäule anzuordnenden Schrauben 3,4 über aie verbindenden Zylinderstücke 20,21 durch Einschrauben der Gewindebereiche in die Bolzen 24 eingesetzt. Die Muttern 41-43 sind noch nicht angezogen, so daß eine ausreichende Bewegung gegeben ist und die Schrauben in die Wirbel einschraubbar sind. In entsprechender Weise ist die Gewindestange 30 über die zugehörigen Zylinderstücke mit den Schrauben 1 und 2 verbunden. Nach dem Einschrauben der Schrauben 1-4 in die zugehörigen Wirbelteile werden die Muttern 37-40 zunächst lose angezogen, so daß der Abstand der gegenüberliegenden Schrauben 1 und 2 und 3 und 4 fixiert ist.

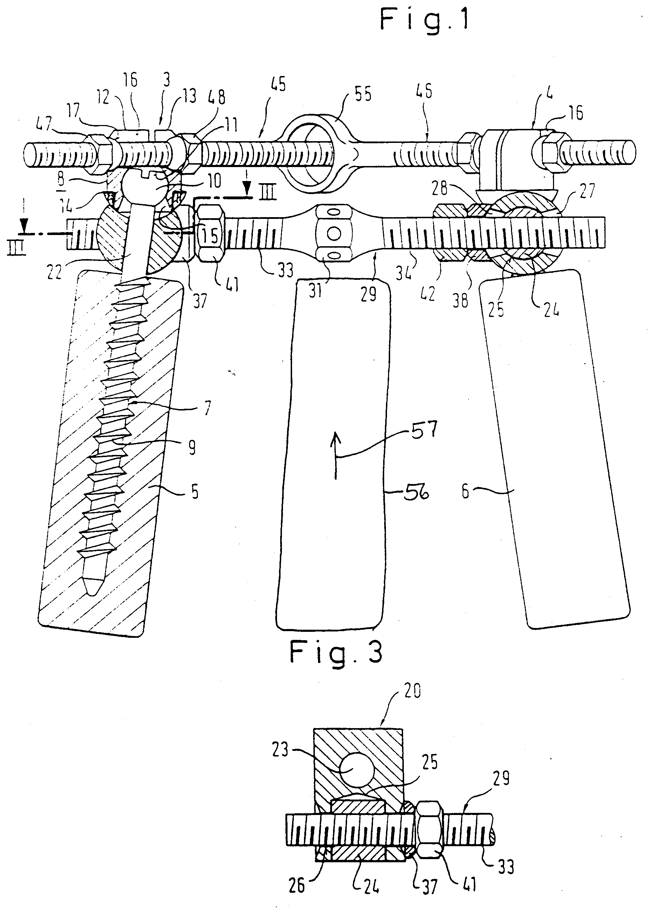

Ferner sind Gewindestangen 45 und 46 vorgesehen. Die Gewindestange 45 ist eine einfache Gewindestange mit zwei Mutternpaaren 47,48; 49,50. Die Mutternpaare weisen einander zugewandte und zur Gewindestange koaxial angeordnete kugelsegmentförmige Abschnitte 51,52 bzw. 53,54 auf. Die jeweiligen Kopfhälften des Aufnahmeteiles 8 weisen koaxial zu dem jeweiligen Aufnahmeschlitz 16 kugelsegmentförmige Versenkungen 17 auf, wie am besten aus Fig. 1 ersichtlich ist. Der Durchmesser der Gewindestange ist so gewählt, daß diese gerade in die Aufnahmeschlitze der Aufnahmeteile der Schrauben 1 und 4 einsetzbar ist. Die Gewindestange 46 unterscheidet sich von der beschriebenen Gewindestange 45 lediglich dadurch, daß sie in ihrem Mittenbereich eine Öse 55 bildenden Abschnitt aufweist. Ansonsten ist die Ausbildung entsprechend so gewählt, daß die Stange in den Aufnahmeschlitz der Schrauben 2 und 3 einsetzbar ist. Die Öse 55 dient zum Hindurchführen der Gewindestange 46 derart, daß die beiden Schrauben quasi in der gleichen Ebene mit den Schrauben 1,4 bzw. 2,3 verbindbar sind. Durch die Verschwenkbarkeit der Aufnahmeteile relativ zu den Schrauben ist das Einsetzen der Gewindestangen 45,46 auf einfache Weise möglich. Nach dem Einsetzen werden die jeweiligen Gewindeschraubenpaare zum vorläufigen Festlegen der Gewindestangen relativ zu den Aufnahmeteilen festgezogen.

Im Betrieb werden zunächst Löcher in das entsprechende Wirbelpaar 5,6 zur Aufnahme der Schrauben 1-4 gebohrt. Dann wird in Abhängigkeit von dem Abstand der den Schrauben 1 und 2 bzw. 3 und 4 entsprechenden Bohrung mit den Gewindestangen 29,30 ein entsprechender Abstand für die Schrauben 1 und 2 bzw. 3 und 4 eingestellt. Anschließend werden die Schrauben 1-4 in die vorbereiteten Löcher eingeschraubt. Im Anschluß daran werden die Gewindestangen 45 und 46 in die zugehörigen Schlitze eingesetzt. Durch Einstellen der Mutternpaare 47,48 bzw. 49,50 wird nun die Winkelstellung der Wirbel 5 und 6 zueinander einjustiert. Eine eventuelle Abstandsveränderung kann gleichzeitig bzw. im Wechselspiel über das Einstellen der Gewindestangen 29 und 30 erreicht werden. Im Anschluß an die gewünschte Ausrichtung werden die Muttern 41-44 und die Mutternpaare 47,48 und 49,50 festgezogen. Durch die oben beschriebene Ausbildung ist einerseits die genaue langenmäßige und winkelmäßige Ausrichtung der Wirbel möglich und kann auch zu einem späteren Zeitpunkmit der gleichen Vorrichtung wiederholt werden. Gleichzeitig wird durch die sich kreuzenden Gewindestangen eine hohe Stabilität gegen Torsionskräfte erzielt. Im Hinblick darauf, daß auch nach der Ausrichtung keine Teile an der Vorrichtung entfernt werden, bleiben diese auch für zukünftige Änderungen der Einstellung voll funktionsfähig.

Wie aus Fig. 1 ersichtlich ist, wird bei der Aufspreizung der Wirbelkörper 5 und 6 der dazwischenliegende Wirbelkörper 56 in Richtung des Pfeiles 57 zu der Seite der Wirbelsäule hin versetzt, an der die aufgespreizten Wirbelkörper den kleineren Abstand voneinander aufweisen, also zu der Korrektur- und Haltevorrichtung hin.

Wie in den Fig. 4 und 5 gezeigt ist, greift deshalb an den beiden Gewindestangen 29 und 30 in dem Bereich des dazwischenliegenden Wirbelkörpers 56 jeweils eine Pedikelschraube 58, 59 an.

Im folgenden wird der Aufbau einer solchen Pedikelschraube beschrieben. Die Pedikelschraube weist einen Gewindeschaftteil 60 mit einem kugelsegment- bzw. kugelförmigen Kopf 61 an seinem freien Ende und einen darauf aufsetzbaren Aufsatz 62 auf. Der Aufsatz weist einen Gewindestab 63 auf, der an seinem einen freien Ende einen Ansatz mit einem sich in axialer Richtung von dem Gewindestab weg erstreckenden pfannenförmigen Lager 64 aufweist. Das pfannenförmige Lager ist bevorzugt als kugelsegmentförmiges Lager ausgebildet, dessen Radius im wesentlichen gleich im Radius des kugelförmigen Teiles des Kopfes 61 ist. Auf seinem dem Lager 64 gegenüberliegenden freien Ende weist der Gewindestab eine Ausnehmung 65 zum Einsetzen eines Schraubendrehers auf. Mit dem Gewindestab ist ein Element 66 verbunden. Dieses weist ein erstes Teil 68 und ein damit verbundenes zweites Teil 69 auf. Das erste Teil ist quaderförmig ausgebildet und weist eine Gewindebohrung mit einem dem Außengewinde des Gewindestabes 63 entsprechenden Gewinde auf und ist über diese Bohrung auf dem Gewindestab aufgeschraubt, wie dies am besten aus den Fig. 6 und 7 ersichtlich ist. Das zweite Teil 69 weist einen in Fig. 8 angedeuteten hervorstehenden Zapfen 70 auf, der in eine entsprechende Bohrung des ersten Teiles 68 eingesetzt ist. Die Achse von Bohrung und Zapfen 70 erstreckt sich quer zur Längsachse des Gewindestabes 63 und bevorzugt senkrecht zu dieser Achse. Der Durchmesser des Zapfens 70 und der entsprechenden Bohrung sind so aufeinander abgestimmt, daß das zweite Teil 69 um die Achse des Stiftes drehbar bzw. schwenkbar ist. Das zweite Teil weist eine U-förmige Auskerbung 71 auf, deren Symetrie-Ebene sich senkrecht zu der Längsachse des Zapfens 70 erstreckt. Die Abmessung der Auskerbung 71 ist so bemessen, daß eine Gewindestange 29 bzw. 30 in die Auskerbung einsetzbar ist. Der Grund der U-förmigen Auskerbung 71 weist ein dem Außengewinde der aufzunehmenden Gewindestange 29 bzw. 30 entsprechendes Innengewinde auf. Gewünschtenfalls kann am oberen Rand der Auskerbung eine sich von der Wandung in die Auskerbung erstreckende Schraube 72, die in Fig. 5 angedeutet ist, vorgesehen sein, die dazu dient, zu verhindern, daß die eingesetzte Gewindestange 29 bzw. 30 sich aus der Auskerbung heraus bewegt. Durch das Innengewinde wird erreicht, daß dieses mit der aufzunehmenden Gewindestange in Eingriff kommt und ein unbeabsichtigtes zeitliches Versetzen des Teiles 62 verhindert wird.

Im Betrieb wird, wie dies am besten aus Fig. 5 ersichtlich ist, auf jeder Seite des mittleren Wirbelkörpers 56 zunächst der Teil aus Gewindeschaftteil 60 und Kopf 61 eingeschraubt. Zu diesem Zweck weist der Kopf auf seiner dem Schaft abgewandten Seite eine sich senkrecht zur Schaftachse erstreckende Ebene mit einer Ausnehmung zum in Eingriff bringen mit einem Schraubendreher auf. Nach dem Eindrehen dieser Schraube wird der Aufsatz 62 mit seinem Lager 64 auf den Kopf 61 aufgesetzt. Durch Drehen des Gewindestabes 63 wird der Abstand des Elementes 66 von dem Kopf 61 so eingestellt, daß die Gewindestangen 29, 30 in dem Grund der Auskerbungen 71 liegen. Das Ausrichten der Achse der Auskerbungen 71 parallel zu den Achsen der Gewindestangen 29, 30 ist einfach möglich, weil das zweite Teil 69 einerseits um die Achse des Zapfens 70 und andererseits zusammen mit dem Gewindestab um desse Längsachse und zusätzlich zusammen mit dem Gewindestab noch innerhalb eines Kegelmantels um den Mittelpunkt des kugelförmigen Abschnittes des Kopfes 61 drehbar bzw. schwenkbar ist. Anschließend wird durch Drehen an den beiden Gewindestäben 63 solange gedreht, bis der Wirbelkörper 56 entgegen der Richtung des Pfeiles 57 zum Ausgleich des durch den Weichteilmantel bewirkten Versatzes in die gewünschte Seitenposition zurückbewegt wird. Alternativ kann das Einstellen natürlich schon vor dem Spreizen so erfolgen, daß ein seitliches Versetzen in Richtung des Pfeiles 57 aufgrund der Wirkung des Weichteilmantels beim Spreizen von vorneherein verhindert wird.

## Patentansprüche

1. Pedikelschraube mit
einem Gewindeschaftteil (60) und einem kugelsegment- bzw. kugelförmigen Kopf (61) gekennzeichnet durch
einem darauf aufsetzbaren Aufsatz (62) mit
einer zu dem Kopf (61) passenden Lagerflache (64) an seinem einen Ende und einem mit dem Aufsatz (62) verbundenen Element (66) zum Verbinden mit einer Stange (29,30),
wobei das Element (66) zum Einstellen des Abstandes zu dem einen Ende auf dem Aufsatz hin- und herbewegbar ist.

2. Pedikelschraube nach Anspruch 1,
dadurch gekennzeichnet, daß der Aufsatz (62) als Gewindestab (63) mit der Lagerfläche (64) an seinem einen Ende ausgebildet ist und das Element (66) eine entsprechende Gewindebohrung (67) aufweist, mit der es mit dem Gewindestab (63) im Eingriff ist.

3. Pedikelschraube nach einem der Ansprüche 1 oder 2,
dadurch gekennzeichnet, daß das Element (66) ein mit dem Aufsatz (62) verbindbares erstes Teil (68) und ein mit der Stange (29,30) verbindbares zweites Teil (69) aufweist und erstes und zweites Teil (68,69) um eine Achse gegeneinander schwenkbar sind.

4. Pedikelschraube nach Anspruch 3,
dadurch gekennzeichnet, daß sich die Achse quer zu der Achse des Gewindestabes (63) erstreckt.

5. Pedikelschraube nach einem der Ansprüche 3 oder 4,
dadurch gekennzeichnet, daß das zweite Teil (69) eine Klaue (71) zur Aufnahme der Stange (29,30) aufweist.

6. Korrektur- und Haltevorrichtung, für die Wirbelsäule, mit jeweils einen Gewindeschaftteil (7) und einen Aufnahmeteil (8) aufweisenden Paaren von Schrauben (1,3; 2,4), einem ersten Paar Von Gewindestangen (45, 46) zum Verbinden von jeweils einer Stange mit zwei der Schrauben an einer ersten Verbindungsstelle an deren Aufnahmeteil (8) sowie einem zweiten Paar von Gewindestangen (29,30), von denen jeweils eine mit zwei der Schrauben an einer gegenüber der ersten Verbindungsstelle zum Gewindeschaftteil (7), hin versetzten zweiten Verbindungsstelle zu verbinden ist, dadurch gekennzeichnet, daß eine Pedikelschraube (58,59) nach einem der Patentansprüche 1 bis 5 vorgesehen ist.

7. Korrektur- und Haltevorrichtung nach Anspruch 6, dadurch gekennzeichnet, daß das erste Paar der Schrauben (1,3) zum Einschrauben auf gegenüberliegenden ersten und zweiten Seiten eines ersten Wirbels und das zweite Paar der Schrauben (2,4) zum Einschrauben auf entsprechenden ersten und zweiten Seiten eines zweiten Wirbels ausgebildet sind.

8. Korrektur- und Haltevorrichtung nach Anspruch 6 oder 7, dadurch gekennzeichnet, daß das zweite Paar Von Gewindestangen mit den auf jeweils einer Seite einer Wirbelsäule einzuschraubenden Schrauben (1,3) verbindbar ist.

9. Korrektur- und Haltevorrichtung nach einem der Ansprüche 6 bis 8, dadurch gekennzeichnet, daß jede Stange des ersten Paares der Gewindestangen (45,46) einerseits mit der für die erste bzw. zweite Seite vorgesehene Schraube (1,3) des ersten Paares und andererseits mit der für die zweite bzw. erste Seite vorgesehenen Schraube (4,2) des zweiten Paares verbindbar ist.

10. Korrektur- und Haltevorrichtung nach Anspruch 9,
dadurch gekennzeichnet, daß eine Stange (46) des ersten Paares eine Öse (55) zum Hindurchführen der zweiten Stange (46) des ersten Paares aufweist.

11. Korrektur- und Haltevorrichtung nach einem der Ansprüche 6 bis 10, dadurch gekennzeichnet, daß die Schrauben (1-4) jeweils einen Gewindeschaffteil (7) und einen damit gelenkig verbundenen Aufnahmeteil (8) zur Aufnahme von Gewindestangen (45,46) aufweisen.

12. Korrektur- und Haltevorrichtung nach Anspruch 11,
dadurch gekennzeichnet, daß Gewindeschaffteil (7) und Aufnahmeteil (8) der Schrauben (1-4) derart über ein Gelenk verbunden sind, daß eine Relativbewegung des Aufnahmeteils (8) gegenüber dem Gewindeschaftteil (7) um eine erste Achse und um eine sich zur ersten Achse quer erstreckende zweite Achse möglich ist.

13. Korrektur- und Haltevorrichtung nach einem der Ansprüche 6 bis 12, dadurch gekennzeichnet, daß die Stangen des zweiten Paares von Gewindestangen (29,30) an ihren jeweiligen zur Verbindung mit den Schrauben dienenden Enden (33-36) entgegengesetzt laufende Gewinde aufweisen.

## Claims

1. Pedicle screw with a threaded shank part (60) and a head (61) in the shape of a sphere or segment of a sphere, characterized by an attachment (62) which can be placed thereon and which at one of its ends has a bearing surface (64) matching the head (61) and has an element (66) which is connected to the attachment (62) and is intended for connection to a bar (29, 30) it being possible for the element (66) to be moved to and fro on the attachment for the purpose of adjusting the distance from the one end.

2. Pedicle screw according to Claim 1, characterized in that the attachment (62) is designed as a threaded rod (63) with the bearing surface (64) at one of its ends, and the element (66) has a corresponding threaded bore (67) via which it engages with the threaded rod (63).

3. Pedicle screw according to either of Claims 1 and 2, characterized in that the element (66) has a first part (68) which can be connected to the attachment (62), and a second part (69) which can be connected to the bar (29, 30), and first part and second part (68, 69) can be pivoted relative to each other about an axis.

4. Pedicle screw according to Claim 3, characterized in that the axis extends transverse to the axis of the threaded rod (63).

5. Pedicle screw according either of Claims 3 and 4, characterized in that the second part (69) has a claw (71) for receiving the bar (29, 30).

6. Correction and retaining device for the spinal column, with pairs of screws (1, 3; 2, 4) each having a threaded shank part (7) and a receiving part (8), a first pair of threaded bars (45, 46) for connecting each bar to two of the screws at a first connection site on the receiving part (8) thereof, and a second pair of threaded bars (29, 30) each one of which is to be connected to two of the screws at a second connection site offset towards the threaded shank part (7) in relation to the first connection site, characterized in that a pedicle screw (58, 59) according to one of Patent Claims 1 to 5 is provided.

7. Correction and retaining device according to Claim 6, characterized in that the first pair of screws (1, 3) are designed for screwing-in on opposite first and second sides of a first vertebra, and the second pair of screws (2, 4) are designed for screwing-in on corresponding first and second sides of a second vertebra.

8. Correction and retaining device according to Claim 6 or 7, characterized in that the second pair of threaded bars can be connected to the screws (1, 3) which are to be screwed-in on each respective side of a spinal column.

9. Correction and retaining device according to one of Claims 6 to 8, characterized in that each bar of the first pair of threaded bars (45, 46) can be connected on the one hand to that screw (1, 3) of the first pair provided for the first or second side, respectively, and on the other hand to that screw (4, 2) of the second pair provided for the second or first side, respectively.

10. Correction and retaining device according to Claim 9, characterized in that one bar (46) of the first pair has an eye (55) through which the second bar (46) of the first pair is guided.

11. Correction and retaining device according to one of Claims 6 to 10, characterized in that the screws (1-4) in each case have a threaded shank part (7) and a receiving part (8) which is connected to the latter in an articulated manner for receiving threaded bars (45, 46).

12. Correction and retaining device according to Claim 11, characterized in that threaded shank part (7) and receiving part (8) of the screws (1-4) are connected via an articulation in such a way that a relative movement of the receiving part (8) with respect to the threaded shank part (7) is possible about a first axis and about a second axis extending transverse to the first axis.

13. Correction and retaining device according to one of Claims 6 to 12, characterized in that the bars of the second pair of threaded bars (29, 30) have, at their respective ends (33-36) serving for connection to the screws, threads which run in opposite directions.

## Revendications

1. Vis pour pedicule vertébral, qui comprend
un élément à tige de boulon filetée (60) et une tête (61) sous forme d'un segment sphérique ou sphérique,
**caractérisée** par
un élément rapporté (62) à poser sur elle, comprenant
une surface d'appui (64) à une de ses extrémité, qui est en correspondance à ladite tête (61), et un élément (66) relié avec ledit élément rapporté (62), pour un raccord à une tige (29, 30),
ledit élément (66) étant mobile de façon va-et-vient sur ladite une extrémité sur ledit élément rapporté, afin d'ajuster la distance.

2. Vis pour pedicule vertébral selon la revendication 1,
**caractérisée** en ce que ledit élément rapporté (62) est configuré sous forme d'une tige filetée (63) à une surface d'appui (64) sur l'une de ses extrémités, et en ce que ledit élément présente un trou taraudé correspondant (67), dans lequel s'engrène ladite tige filetée (63).

3. Vis pour pedicule vertébral selon une quelconque des revendications 1 ou 2,
**caracterisée** en ce que ledit élément rapporté (66) comprend une première partie (68) à raccorder audit élément rapporté (62), et une deuxième partie (69) à raccorder à ladite tige (29, 30), et en ce que ladite première partie et ladite deuxième partie (68, 69) sont disposées de façon à être pivotée l'une relativement à l'autre.

4. Vis pour pedicule vertébral selon la revendication 3,
**caractérisée** en ce que l'axe s'étend en travers à l'axe de ladite tige filetée (63).

5. Vis pour pedicule vertébral selon une quelconque des revendications 3 ou 4,
**caractérisée** en ce que ladite deuxième partie (69) comprend un clabot (71) à recevoir ladite tige (29, 30).

6. Dispositif de correction et de maintien pour la colonne vertébrale, comprenant des paires respectives de vis (1, 3; 2, 4), dont chacune a un élément à tige filetée (7) et un élément récepteur (8), une première paire de tiges filetées (45, 46) à raccorder respectivement une tige moyennant de deux desdites vis à un premier point de raccordement à son élément récepteur (8), ainsi qu'une deuxième paire de tiges filetées (29, 30), dont respectivement une est prévue à être raccordée, moyennant deux desdites vis, à un deuxième point de raccordement, qui se trouve en déport relativement audit premier point de raccordement vers ledit élément à tige filetée (7), **caractérisé** en ce qu'une vis (58, 59) pour pedicule vertébral selon une quelconque des revendications 1 à 5 est prévue.

7. Dispositif de correction et de maintien selon la revendication 1, **caractérisé** en ce que ladite première paire de vis (1, 3) est configurée à être visser sur le premier et le deuxième côté opposés d'une première vertèbre, et la deuxième paire de vis (2, 4) est configurée à être vissée sur des premier et deuxième côtés correspondants d'une deuxième vertèbre.

8. Dispositif de correction et de maintien selon les revendications 6 ou 7, **caractérisé** en ce que ladite deuxième paire de tiges filetées est prévue à être raccordée auxdites vis (1, 3) à visser de respectivement un côté d'une colonne vertébrale.

9. Dispositif de correction et de maintien selon une quelconque des revendications 6 à 8,
**caractérisé** en ce que chaque tige de ladite première paire de tiges filetées (45, 46) est prévue, d'un côté, à être raccordée à ladite vis (1, 3) de ladite première paire, qui est prévue pour le premier ou respectivement le deuxième côté, et, d'autre côté, à être raccordée à ladite vis (4, 2) de ladite deuxième paire, qui est prévue pour le deuxième ou respectivement premier côté.

10. Dispositif de correction et de maintien selon la revendication 9, **caractérisé** en ce qu'une tige (46) de ladite première paire présente un oeillet (55) pour le passage de ladite deuxième tige (46) de la première paire.

11. Dispositif de correction et de maintien selon une quelconque des revendications 6 à 10,
**caractérisé** en ce que chacune desdites vis (1 à 4) comprend un élément à tige filetée (7) et un élément récepteur (8) y articulé afin de recevoir des tiges filetées (45, 46).

12. Dispositif de correction et de maintien selon la revendication 11, **caractérisé** en ce que ledit élément à tige filetée (7) et ledit élément récepteur (8) desdites vis (1 à 4) sont reliés par une articulation de façon à permettre un mouvement relatif dudit élément récepteur (8), relativement audit élément à tige filetée (7), autour d'un premier axe et autour d'un deuxième axe qui s'étend en travers audit premier axe.

13. Dispositif de correction et de maintien selon une quelconque des revendications 6 à 12,
**caractérisé** en ce que lesdites tiges de ladite deuxième paire de tiges filetées (29, 30) présentent, à leurs extrémités respectives (33-36), qui servent au raccord aux vis, des filetages qui tourne en sens inverse.
